## Europäisches Patentamt
## European Patent Office
## Office européen des brevets

(11) Veröffentlichungsnummer : **0 132 570**
**B1**

(12) **EUROPÄISCHE PATENTSCHRIFT**

(45) Veröffentlichungstag der Patentschrift :
03.12.86

(21) Anmeldenummer : 84106857.0

(22) Anmeldetag : 15.06.84

(51) Int. Cl.⁴ : **C 11 C 1/10, C 07 C 51/44**

(54) Verbessertes Verfahren zur schonenden Destillation von Fettsäuren.

(30) Priorität : 23.06.83 DE 3322535

(43) Veröffentlichungstag der Anmeldung :
13.02.85 Patentblatt 85/07

(45) Bekanntmachung des Hinweises auf die Patenterteilung : 03.12.86 Patentblatt 86/49

(84) Benannte Vertragsstaaten :
BE DE FR IT NL SE

(56) Entgegenhaltungen :
DE-A- 1 944 473
DE-A- 3 129 883

(73) Patentinhaber : Henkel Kommanditgesellschaft auf Aktien
Postfach 1100 Henkelstrasse 67
D-4000 Düsseldorf-Holthausen (DE)

(72) Erfinder : Jeromin, Lutz, Dr.
Am Bandsbusch 88
D-4010 Hilden (DE)
Erfinder : Johannisbauer, Wilhelm, Dr.
Erich-Kästner-Strasse 26
D-4006 Erkrath 1 (DE)
Erfinder : Thorausch, Klaus
Graf-Recke-Strasse 140
D-4000 Düsseldorf (DE)
Erfinder : Skrapac, Franjo
7, 5th Avenue
6050 Perth Erth/Western (AU)
Erfinder : Hartmann, Helmut, Dr.
Kolpingstrasse 4
D-4018 Langenfeld (DE)
Erfinder : Hentschel, Karl
Boschstrasse 40
D-4000 Düsseldorf 13 (DE)
Erfinder : Michel, Otto
Am Bendenbusch 11
D-4018 Langenfeld (DE)

**Beschreibung**

Die Erfindung betrifft ein verbessertes Verfahren zur schonenden Destillation von Fettsäuren mit 6 bis 24 C-Atomen, die durch Spaltung von natürlichen Fetten und Ölen oder durch synthetische Verfahren, wie z. B. Paraffinoxidation, erhalten werden, durch Trocknen des Rohprodukts in der Wärme unter reduziertem Druck, fraktioniertes Verdampfen des getrockneten und erwärmten Rohprodukts in Fallfilmverdampfern, gegebenenfalls unter Zufuhr überhitzen Wasserdampfs, und Niederschlagen der verschiedenen Dampffraktionen in Kondensatoren.

Fettsäuren aus der technischen Spaltung von Fetten natürlichen Ursprungs oder von Ölen enthalten neben einem Gemisch freier Fettsäuren noch ungespaltene Fettsäureglyceride sowie Fettbegleitstoffe, wie Sterine, Phosphatide, polymerisierte Fettsäuren und sonstige Zersetzungsprodukte sowie weitere Verunreinigungen. Zur Abtrennung der unerwünschten Begleitstoffe, die zum Teil für eine unerwünschte Färbung, eine geringe Farbstabilität sowie einen unangenehmen Geruch der Produkte verantwortlich sind, werden Spaltfettsäuren destilliert.

Die Destillation von Fettsäuren wird großtechnisch schon seit langer Zeit durchgeführt. Die ersten Fettsäure Destillationsanlagen bestanden aus direkt mit Rauchgasen beheizten Destillationsblasen und arbeiteten bei Normaldruck. Da hierbei die zu destillierenden Rohfettsäuren sehr hohen Temperaturen ausgesetzt waren und dadurch teilweise Zersetzung bzw. Polymerisation eintrat, erhielt man Destillate, die mehr oder weniger stark gefärbt waren und sich zudem durch unangenehmen Geruch auszeichneten. Die Qualität der so hergestellten Destillate entspricht nicht mehr den heutigen Ansprüchen.

Um aus rohen Spaltfettsäuren qualitativ hochwertige Destillate zu erhalten, muß die Destillation bei niedrigen Destillationstemperaturen und damit hohem Vakuum sowie möglichst kurzer Verweilzeit des Rohproduktes durchgeführt werden. Technisch arbeitet man hauptsächlich bei 2 bis 10 mbar und Temperaturen bis etwa 260 °C (Ullmanns Enzyklopädie der technischen Chemie, Band 11, Seite 533ff, Verlag Chemie Weinheim, 1976).

Öle und Fettsäuren können durch Adsorptionsverfahren gereinigt und entfärbt werden. Die am meisten angewendeten Adsorptionsmittel sind natürliche und/oder aktivierte Bleicherden. Vereinzelt wird auch Aktivkohle verwendet. Nachteilig bei diesen Adsorptionsmethoden ist es, daß die eingesetzen Bleichstoffe relativ teuer sind und eine gewisse Menge der Produktfettsäuren aufnehmen, was die Ausbeuten unter Umständen drastisch vermindert.

Unter den destillativen Reinigungsverfahren, die heute fast alle mit Zugabe von mehr oder weniger Wasserdampf zur Erniedrigung des Partialdruckes und damit des Siedepunktes der Fettsäuren arbeiten, gibt es eine Reihe von Verfahren, die bei der Destillation weitere Zusatzstoffe benutzen. Beschrieben wurde die Verwendung von Bortrifluorid (US-A-2 583 028), aromatischen Carbonsäuren (DE-B-19 44 473), aromatischen oder aliphatischen Aminoverbindungen (JP-A-7408/78 bzw. US-A-3 471 536), sowie reduzierenden Metallen und ihren Hydroxiden (DE-A-29 39 242). Alle diese Verfahren, die auf einer destillativen Trennung unter Zugabe der genannte Stoffe beruhen, haben den Nachteil von mehr oder weniger großen Produktverlusten. Außerdem treten mitunter Korrosionsprobleme an den bei hoher Temperatur mit den Rohmischungen in Kontakt kommenden Anlagenteilen auf.

Aufgabe der vorliegenden Erfindung war es, ein Verfahren zur Verfügung zu stellen, in dem Fettsäuren natürlichen Ursprungs bei niedrigen Temperaturen und kurzer Verweilzeit in einer einzigen Stufe mit hohen Ausbeuten von unerwünschten Verunreinigungen befreit und destillativ voneinander getrennt werden können. Die Produkte sollten reproduzierbare Farbqualität, hohe Farbstabilität und besseren Geruch aufweisen als die nach den bekannten Verfahren gewonnenen Fettsäurefraktionen.

Demgemäß betrifft die Erfindung ein verbessertes Verfahren zur schonenden Destillation von Fettsäuren mit 6 bis 24 C-Atomen, die durch Spaltung von natürlichen Fetten und Ölen oder durch synthetische Verfahren, wie z. B. Paraffinoxidation, erhalten werden, durch Trocknen des Rohprodukts in der Wärme unter reduziertem Druck, fraktioniertes Verdampfen des getrockneten und erwärmten Rohprodukts in Fallfilmverdampfern, gegebenenfalls unter Zufuhr überhitzen Wasserdampfs, und Niederschlagen der verschiedenen Dampffraktionen in Kondensatoren, das dadurch gekennzeichnet ist, daß

a) der Trocknungsprozeß bei 60 bis 80 °C und 90 bis 100 mbar Druck durchgeführt wird,

b) zum Verdampfen der niedrigsiedenden Fraktion das getrocknete Rohprodukt auf 212 bis 224 °C bei einem Druck von 5 bis 20 mbar erhitzt wird,

c) der flüssige Rest ausdestilliert wird,

d) die Dämpfe eine Rektifikationskolonne durchströmen, deren Einbauten bei einer vergleichbaren Luftgeschwindigkeit von mindestens 2 m/s einen Druckverlust von höchstens 1 mbar/m Packungshöhe aufweisen, wobei die Kolonne mindestens 4 Trennstufen hat,

e) die Dämpfe in zwei Kondensatoren niedergeschlagen werden,

f) das Kondensat aus dem ersten Kondensator als Rücklauf auf den Kopf der Rektifikationskolonne gegeben wird,

g) eine kleine Menge Vorlauf aus dem zweiten Kondensator abgezogen wird,

h) der Hauptlauf als Seitenabzug aus der Kolonne abgezogen wird,

2

i) ein Rücklauf aus dem Seitenabzug unterhalb der Abzugsstelle des Hauptlaufs zurückgegeben wird und

j) eine kleine Menge Rückstand aus dem im Kreislauf betriebenen zweiten Fallfilmverdampfer laufend abgezogen wird.

Die Rektifikation und Reinigung der eingesetzten Fettsäuren findet — nach Trocknung und Vorwärmung — in einer einzigen Rektifikationskolonne statt. Das Sumpfprodukt wird auf einem mit der Kolonne verbundenen Fallfilmverdampfer ausdestilliert. Am Kopf wird eine durch partielle Kondensation abgetrennte Leichtsiederfraktion von dunkelroter Farbe und unangenehmem Geruch abgezogen. Es handelt sich hierbei unter anderem um niedermolekulare Kohlenwasserstoffe, Methylketone und Aldehyde. Die Trennung der Rohfettsäure von der Schmutz- und Geruchsfraktion findet in einem einzigen Durchlauf statt.

Das vorgeschlagene Verfahren liefert Fettsäuren mit guter Farbqualität und -stabilität. Gegenüber den nach dem Stand der Technik bekannten Verfahren werden jedoch in höherer Ausbeute Produkte erhalten, die sich durch einen schwachen, aber angenehmen Geruch auszeichnen.

Die in dem erfindungsgemäßen Verfahren eingesetzte Rektifikationskolonne ist in zwei von ihrer Funktion her unterschiedliche Abschnitte eingeteilt. Der obere Teil zwischen Kopf und Seitenabzug dient der Aufkonzentrierung der Leichtsieder am Kopf und der Kondensation des Hauptlaufs. Der untere Teil ermöglicht die Abtrennung der Schwersieder und Abtrennung von aus den Fallfilmverdampfern mitgerissenen Tröpfchen.

Für die Anreicherung der Leichtsieder am Kopf und der Schwersieder im Sumpf ist eine bestimmte Trennstufenzahl erforderlich. Zur Verwirklichung dieser Trennstufenzahl sind Einbauten in Form von Böden oder Füllkörperschüttungen üblich. Diese Einbauten verursachen jedoch einen Druckverlust in der Kolonne, der zu einem höheren Sumpfdruck und damit zu einer höheren Sumpftemperatur mit nachteiligen Folgen für Farbe, Geruch und Ausbeute der destillierten Fettsäure führt.

Durch die Entwicklung von Einbauten mit geringem Druckverlust bei gleichzeitig guter Trennwirkung ist das Verfahren gemäß der Erfindung erst möglich geworden. Unter druckverlustarmen Einbauten sind bei diesem Verfahren Einbauten zu verstehen, die bei einer vergleichbaren Luftgeschwindigkeit von 2 m/s einen Druckverlust von höchstens 1 mbar/m aufweisen. Dazu zählen metallische Pallringe, insbesondere aber Gewebepackungen oder regelmäßige Blechpackungen, wie etwa BX-Packung® und Mellapak®.

Im übrigen entspricht der Aufbau der Rektifikationskolonne den üblichen Konstruktionen, bestehend aus : Auflagerost, Packung, Abdeckrost, Verteilerboden (Hauptlauf-Rücklauf) und Sammelboden (Hauptlauf) im unteren Abschnitt und Auflagerost, Packung, Abdeckrost, Verteilerboden (Vorlauf-Rücklauf) im oberen Abschnitt.

Roste und Böden sind strömungsgünstig ausgeführt, um auch hier den Druckverlust zu minimieren.

Aus dem beiliegenden Fließchema geht der Produktgang des erfindungsgemäßen Verfahrens im einzelnen hervor. Die Ziffern in dem Fließchema haben folgende Bedeutung :

1 Entgaser
2 Einsatzpumpe
3 Vorwärmer
4 erster Fallfilmverdampfer
5 unterteilte Blase
6 Sumpfteil der Blase
7 Sumpfumwälzpumpe
8 Zweigleitung zum Kopf 9
9 zweiter Fallfilmverdampfer
10 Rückstandsteil der Blase
11 Rückstandspumpe
12 Rückstandskühler
13 Dampferhitzer
14 Rektifikationskolonne
15 oberste Trennstufe
16 übrige Trennstufen
17 erster Kondensator
18 zweiter Kondensator
19 Pumpenvorlage
20 Pumpe für den Vorlauf-Rücklauf
21 Erhitzer für den Vorlauf-Rücklauf
22 Dampfleitung
23 Hauptlauf-Leitung
24 Pumpenvorlage
25 Pumpe für den Hauptlauf-Abzug und Hauptlauf-Rücklauf
26 Kühler
27 Erhitzer für den Hauptlauf-Rücklauf
28 Pumpe für den Vorlauf-Abzug

29 Vakuumpumpe
30 Wasserringpumpe

Das Rohfettsäuregemisch wird aus dem Kesselwagen in den Entgaser 1 gefördert. Bei einem Druck von 90 bis 110 mbar und 60 bis 80 °C werden gelöste Gase aus der Fettsäure entfernt ; gleichzeitig findet eine Verdampfung des im Rohgemisch enthaltenen Wassers statt. Die freiwerdenden Gase und Dämpfe werden über eine Vakuumleitung abgesaugt, die zur Wasserringpumpe 30 führt.

Die Einsatzpumpe 2 drückt die Rohfettsäure durch einen Vorwärmer 3, der mit Dampf von 4 bar beheizt wird. Der Einsatz verläßt den Vorwärmer 3 mit einer Temperatur von 140 bis 150 °C und fließt von dort in den mit Diphyl® beheizten Fallfilmverdampfer 4. Diphyl® ist eine flüssige Mischung aus Biphenyl und Diphenylether oder Ditolyletherisomeren, das als Wärmeübertragungsmittel im Bereich von 200 bis 400 °C benutzt wird. Bei einer Temperatur von 212 bis 224 °C und einem Druck von 5 bis 20 mbar verdampft im Fallfilmverdampfer 4 ein großer Teil der Fettsäuren des Gemisches.

Der im Fallfilmverdampfer 4 nicht verdampfende Anteil des Einsatzes fließt in den Bodensumpf 6 der unterteilten Blase 5 und wird von dort durch die Sumpfumwälzpumpe 7 zum Kopf des Fallfilmverdampfers 4 zurückgepumpt. Aus dem Kreislauf des Fallfilmverdampfers 4 wird laufend eine relativ kleine Menge Flüssigkeit aus dem Sumpf 6 über eine Niveauregulierung und über die Leitung 8 zum Kopf des zweiten Fallfilmverdampfers 9 gepumpt. Die Flüssigkeit im Rückstandsteil 10 der geteilten Blase 5 wird über eine Niveauregulierung und die Sumpfumwälzpumpe 11 zum Kopf des zweiten Fallfilmverdampfers 9 gepumpt, der bei einem Druck von 5 bis 20 mbar und — je nach der Art des eingesetzten Fettsäuregemisches und der gewünschten Rest-Säurezahl im Rückstand — einer Temperatur von 220 bis 260 °C arbeitet. Der Fallfilmverdampfer 9 wird ebenfalls im Umlauf betrieben. Aus dem Kreislauf des Fallfilmverdampfers 9 wird laufend eine relativ kleine Menge Rückstand über eine Niveauregulierung und den Kühler 11 abgezogen.

Da die Rektifikationskolonne 14 mit druckverlustarmen Einbauten ausgerüstet ist, ist der Sumpfdruck bereits so niedrig, daß im Prinzip auf eine weitere Partialdruckerniedrigung verzichtet werden kann, um Produkte mit der gewünschten Farb- und Geruchsqualität zu erreichen. Wird eine weitere Verbesserung der Produktqualität gewünscht, so kann dies durch Zugabe von überhitztem Wasserdampf in einer Menge von Maximal 10 Gewichtsprozent, bezogen auf das Einsatzmaterial, erreicht werden. Die Zugabe von überhitztem Wasserdampf dient vor allen Dingen der Desodorierung der Fettsäuren, wenn diese erforderlich ist. Falls die Anwesenheit von Wasserdampf gewünscht wird, kann dieser aus einem Dampferhitzer 13 am Kopf des zweiten Fallfilmverdampfers 9 eingeführt werden.

Die beiden Fallfilmverdampfer werden mit konstanter Diphenyl-Vorlauftemperatur betrieben ; die Austrittstemperaturen der Fettsäure werden so geregelt, daß eine Überhitzung der Fettsäuren vermieden wird.

Die in den beiden Verdampfern 4 und 9 verdampften Fettsäuren durchströmen gemeinsam die Rektifikationskolonne 14. Die Kolonne ist mit Pallringen oder regelmäßigen Kolonnenpackungen gefüllt und hat mindestens 4 Trennstufen, die oberste Trennstufe 15 und die übrigen Trennstufen 16. Die Kolonne sitzt auf einer Blase 5, die durch ein Blech in einen Sumpfteil 6 und einen Rückstandsteil 10 unterteilt ist, so daß Sumpf und Rückstand nicht vermischt werden. Die am Kolonnenkopf anfallenden Dämpfe werden in zwei hintereinander geschalteten Kondensatoren 17 und 18 niedergeschlagen.

Im Kondensator 17 wird der größte Teil der Dämpfe kondensiert und mit einer bestimmten Temperatur über eine Pumpenvorlage 19, eine Rücklaufpumpe 20 und einen Erhitzer 21 als Vorlauf-Rücklauf zum Kopf der Rektifikationskolonne 14 gefördert.

Unterhalb des Kolonnenkopfes werden in der obersten Trennstufe 15 die leicht siedenden Dämpfe angereichert und der Hauptlauf kondensiert. Der Hauptlauf wird etwa eine Trennstufe unterhalb des Kolonnenkopfes über einen Seitenabzug abgezogen und fließt über die Leitung 23 in die Pumpenvorlage 24. Von dort kann der Hauptlauf mit der Pumpe 25 über einen Kühler 26 aus der Anlage gefahren werden. Ein kleiner Teil des Hauptlaufes wird als Rücklauf über den Wärmeaustauscher 27 unterhalb des Seitenabzugs auf die unteren Trennstufen 16 der Kolonne 14 gegeben. Im unteren Teil 16 der Kolonne 14 werden aus der Blase mitgerissene Flüssigkeitstropfen abgeschieden und schwer siedende Farbträger durch Rektifikation abgetrennt.

Im Kondensator 18 wird der restliche Teil der Dämpfe kondensiert und über die Pumpe 28 abgezogen.

Zur Anlage gehört ferner eine Vakuumpumpe 29, bestehend aus einem dreistufigen Dampfstrahlaggregat mit Kondensatoren, und eine Wasserringpumpe 30.

Das Verfahren gemäß der vorliegenden Erfindung wurde getestet an Fettsäuregemischen, die aus der Spaltung von Talg (Beispiel 1) gewonnen wurden, und an Rückstandsfettsäure aus der Fettspaltung der ersten Destillationsrückstände (Beispiel 2). Olein-Fettsäuren aus Talg bestehen zu ca. 85 % aus Öl- bzw. Stearinsäure, Rückstandsfettsäure besteht zu ca. 66 % aus Öl- bzw. Stearinsäure, der Rest entfällt in der Hauptsache auf Palmitin- und Palmitoleinsäure.

Die Farben der Fettsäuren wurden nach Lovibond in einer 5 1/4 "-Küvette gemessen.

Zur Beurteilung der Farbqualität wurden Hitzetests durchgeführt. Dazu wurden Proben der nach dem erfindungsgemäßen Verfahren gewonnenen Produkte in einem 5 cm hoch gefüllten 125 ml-Weithals-Becherglas gleichbleibend bei 60 °C 72 h lang im Wärmeschrank aufbewahrt. Die Beurteilung der Farbe

4

erfolgte wie angegeben.

Der Geruch der Proben wurde qualitativ im Vergleich zu Proben aus den herkömmlichen Produktionsanlagen beurteilt.

### Beispiel 1

| Einsatz: | Talg-Fettsäure | |
|---|---|---|
| Druck | 3 mbar | Kolonnenkopf |
| | 9 mbar | Kolonnensumpf |
| Temperatur | 216°C | Fallfimverdampfer 4 |
| | 220°C | " 9 |
| Wasserdampf | 8 % | vom Einsatz |
| Vorlaufabzug | 2 % | vom Einsatz |
| Hauptlaufrücklauf | 20 % | vom Hauptlauf |
| Farbe | 4 gelb | (Lovibond 5 1/4") |
| | 0,6 rot | |
| Säurezahl des Rückstands | 85 | |
| Geruch Hauptlauf | schwach, angenehm | |

### Beispiel 2

| Einsatz: | Rückstand-Fettsäure | |
|---|---|---|
| Bedingungen wie Beispiel 1 außer | | |
| Hauptlaufrücklauf | 50 % | vom Hauptlauf |
| Farbe | 10 gelb | (Lovibond 5 1/4 ") |
| | 1,6 rot | |

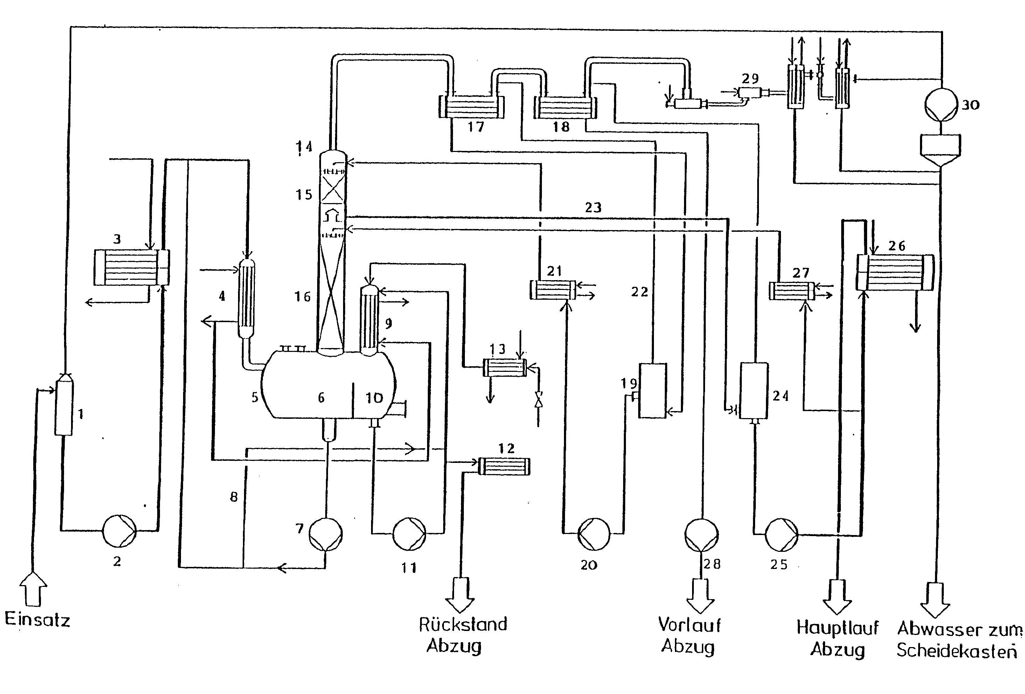

**Patentansprüche**

1. Verfahren zur schonenden Destillation von Fettsäuren mit 6 bis 24 C-Atomen, die durch Spaltung von natürlichen Fetten und Ölen oder durch synthetische Verfahren erhalten werden, durch Trocknen des Rohprodukts in der Wärme unter reduziertem Druck, fraktioniertes Verdampfen des getrockneten und erwärmten Rohprodukts in Fallfilmverdampfern, gegebenenfalls unter Zufuhr überhitzen Wasserdampfs, und Niederschlagen der verschiedenen Dampffraktionen in Kondensatoren, dadurch gekennzeichnet, daß

a) der Trocknungsprozeß bei 60 bis 80 °C und 90 bis 100 mbar Druck durchgeführt wird,

b) zum Verdampfen der niedrigsiedenden Fraktion das getrocknete Rohprodukt auf 212 bis 224 °C bei einem Druck von 5 bis 20 mbar erhitzt wird,

c) der flüssige Rest ausdestilliert wird,

d) die Dämpfe eine Rektifikationskolonne durchströmen, deren Einbauten bei einer vergleichbaren Luftgeschwindigkeit von mindestens 2 m/s einen Druckverlust von höchstens 1 mbar/m Packungshöhe aufweisen, wobei die Kolonne mindestens 4 Trennstufen hat,

    e) die Dämpfe in zwei Kondensatoren niedergeschlagen werden,

    f) das Kondensat aus dem ersten Kondensator als Rücklauf auf den Kopf der Rektifikationskolonne gegeben wird,

    g) eine kleine Menge Vorlauf aus dem zweiten Kondensator abgezogen wird,

    h) der Hauptlauf als Seitenabzug aus der Kolonne abgezogen wird,

    i) ein Rücklauf aus dem Seitenabzug unterhalb der Abzugsstelle des Hauptlaufs zurückgegeben wird und

    j) eine kleine Menge Rückstand aus dem im Kreislauf betriebenen zweiten Fallfilmverdampfer laufend abgezogen wird.

2. Verfahren nach Anspruch 1, dadurch gekennzeichnet, daß bis 10 % überhitzter Wasserdampf in den zweiten Fallfilmverdampfer eingeleitet wird.

3. Verfahren nach einem der Ansprüche 1 oder 2, dadurch gekennzeichnet, daß von den wenigstens vier Trennstufen der Rektifikationskolonne eine oberhalb des Hauptdestillatabzuges und die übrigen Trennstufen unterhalb des Hauptdestillatabzuges angebracht sind.

4. Verfahren nach einem der Ansprüche 1 bis 3, dadurch gekennzeichnet, daß der Ablauf aus dem zweiten Kondensator in einer Menge von 0,5 bis 5 %, bezogen auf den Zulauf, als Vorlauf abgezogen wird.

## Claims

1. A process for the mild distillation of $C_6$-$C_{24}$ fatty acids obtained by splitting of natural fats and oils or by synthetic methods by drying the crude product under heat under reduced pressure, subjecting the dried and heated crude product to fractional evaporation in falling film evaporators, optionally with introduction of superheated steam, and precipitating the various vapor fractions in condensers, characterized in that

    a) the drying process is carried out at 60 to 80 °C/90 to 100 mbar pressure,

    b) for evaporation of the low-boiling fraction, the dried crude product is heated to 212-224 °C under a pressure of from 5 to 20 mbar,

    c) the liquid residue is thoroughly distilled.

    d) the vapors flow through a rectification column of which the flights show a pressure loss of at most 1 mbar/m packing height at a comparable air velocity of at least 2 m/s, the column having at least 4 separation stages,

    e) the vapors are precipitated in two condensers,

    f) the condensate from the first condenser is delivered as return flow to the head of the rectification column,

    g) a small quantity of first runnings is run off from the second condenser,

    h) the main runnings are run off as a sidestream from the column,

    i) a return flow from the sidestream is fed back below the point at which the main runnings are removed and

    j) a small quantity of residue from the second, circulation-type falling film evaporator is continuously removed.

2. A process as claimed in Claim 1, characterized in that up to 10 % superheated steam is introduced into the second falling film evaporator.

3. A process as claimed in Claim 1 or 2, characterized in that, of the at least four separation stages of the rectification column, one is arranged above the main distillate outlet while the other are arranged below the main distillate outlet.

4. A process as claimed in any of Claims 1 to 3, characterized in that the effluent from the second condenser is run of as first runnings in a quantity of from 0.5 to 5 %, based on the influent.

## Revendications

1. Procédé pour la distillation modérée d'acides gras contenant 6 à 24 atomes de carbone que l'on obtient par dissociation d'huiles et de graisses naturelles ou par des procédés de synthèse, par séchage du produit brut à chaud et sous pression réduite, par évaporation fractionnée du produit brut séché et chauffé dans des évaporateurs à pellicule descendante, éventuellement avec apport de vapeur d'eau surchauffée et précipitation des différentes fractions de vapeur dans des condenseurs, ce procédé étant caractérisé en ce que :

    a) on effectue le processus de séchage à 60-80 °C et sous une pression de 90 à 100 mbars,

    b) pour évaporer la fraction à bas point d'ébullition, on chauffe le produit brut séché à 212-224 °C sous une pression de 5 à 20 mbars,

    c) on sépare le résidu liquide par distillation,

    d) les vapeurs circulent à travers une colonne de rectification dont les pièces rapportées ont, à une vitesse d'air comparable d'au moins 2 m/s, une perte de charge au maximum de 1 mbar/m de hauteur de garnissage, la colonne comportant au moins 4 étages de séparation,

e) les vapeurs sont précipitées dans deux condenseurs,

f) le condensat du premier condenseur est acheminé comme reflux en tête de la colonne de rectification,

g) on retire une petite quantité de têtes du deuxième condenseur,

h) on retire latéralement la fraction principale de la colonne,

i) on recharge un reflux de l'évacuation latérale en dessous du point d'évacuation de la fraction principale et

j) on retire continuellement une faible quantité de résidu du deuxième évaporateur à pellicule descendante fonctionnant en circuit.

2. Procédé selon la revendication 1, caractérisé en ce qu'on introduit jusqu'à 10 % de vapeur d'eau surchauffée dans le deuxième évaporateur à pellicule descendante.

3. Procédé selon une des revendications 1 ou 2, caractérisé en ce que, parmi au moins les quatre étages de séparation de la colonne de rectification, on en adapte un au-dessus de l'évacuation du distillat principal et les autres étages de séparation, en dessous de l'évacuation du distillat principal.

4. Procédé selon une des revendications 1 à 3, caractérisé en ce que l'écoulement hors du deuxième condenseur est évacué comme fraction de tête en une quantité de 0,5 à 5 %, calculé sur l'alimentation.